Europäisches Patentamt

European Patent Office   (11) Numéro de publication: **0 210 483**
**B1**
Office européen des brevets

⑫ **FASCICULE DE BREVET · EUROPEEN**

(45) Date de publication du fascicule du brevet: (51) Int. Cl.⁴: **A61K 7/00, C11C 1/00**
**18.04.90**

(21) Numéro de dépôt: **86109345.8**

(22) Date de dépôt: **09.07.86**

(54) **Composition de corps gras d'origine végétale pour usage cosmétique.**

(30) Priorité: **17.07.85 FR 8510976**

(43) Date de publication de la demande:
**04.02.87 Bulletin 87/6**

(45) Mention de la délivrance du brevet:
**18.04.90 Bulletin 90/16**

(84) Etats contractants désignés:
**AT BE CH DE FR GB IT LI LU NL SE**

(56) Documents cités:
**DE-A- 2 232 323**
**DE-A- 2 250 866**

(73) Titulaire: **Bosserelle, Micheline, 29, Rue Pierre Nicole, F-75005 Paris(FR)**

(72) Inventeur: **Bosserelle, Micheline, 29, Rue Pierre Nicole, F-75005 Paris(FR)**

(74) Mandataire: **Casalonga, Axel et al, BUREAU D.A. CASALONGA - JOSSE Morassistrasse 8, D-8000 München 5(DE)**

ACTORUM AG

## Description

L'invention a pour objet une composition de corps gras d'origine végétale destinée à être utilisée dans des compositions cosmétiques.

On sait que les compositions cosmétiques et plus particulièrement les compositions cosmétiques pour le traitement de la peau et des cheveux renferment des composants gras. Divers corps gras d'origine animale ont été utilisés dans les compositions cosmétiques, notamment les huiles de vison, de marmotte et de tortue marine.

Cependant ces corps gras d'origine animale présentent divers inconvénients.

Ils sont souvent souillés de sang et d'autre impuretés et rancissent rapidement. En outre à côté des constituants ayant une bonne compatibilité avec l'épiderme, ces corps gras d'origine animale renferment également des constituants, notamment des acides gras à chaîne courte, qui irritent la peau.

Enfin la production de ces corps gras d'origine animale nécessite la mise à mort d'un grand nombre d'animaux ce qui risque de perturber l'équilibre écologique.

Un besoin existe par conséquent de remplacer les corps gras d'origine animale destinés aux compositions cosmétiques par des corps gras d'origine végétale.

La demanderesse a découvert qu'une composition de corps gras d'origine végétale, appelée ci-après CHELONINE convient pour remplacer avantageusement les corps gras d'origine animale et plus particulièrement l'huile de tortue marine dans les utilisations cosmétiques.

La chelonine présente par rapport aux huiles animales et plus particulièrement par rapport à l'huile de tortue marine l'avantage d'une meilleure compatibilité avec l'épiderme, par suite de l'absence d'acides gras ayant 12 atomes de carbone ou moins de 12 atomes de carbone et par suite de la présence d'au moins un ester d'acides gras ayant au total 40 atomes de carbone. La présence de cet ester en $C_{40}$ lui assure en outre une meilleure stabilité à l'oxydation et par suite une meilleure conservation.

La chelonine est composée de 85 à 94% et de préférence de 88 à 92% de triglycérides d'acides gras, de 7 à 14% et de préférence de 8,5 à 11,5% et plus préférablement de 10% d'ester d'acides gras ayant au total 40 atomes de carbone, de 0,01 à 1% et de préférence de 0,1 à 0,5% de vitamines et éventuellement de 0,5 à 1,5% d'antioxydant.

La composition moyenne des acides gras des triglycérides, exprimée en % en poids est la suivante :
— acide myristique 0,35–0,7% et de préférence 0,45–0,6%
— acide palmytique 68–76% et de préférence 70–74%
— acide stéarique 4–7,5% et de préférence 5,5–6,7%
— acide linoléïque 8–10,5% et de préférence 8,5–9,5%
— acide linolénique 0,5–2% et de préférence 0,6–1,6%
— acide oléique 8–14% et de préférence 10,3–12,3%.

Dans les triglycérides d'acides gras la proportion des acides gras insaturés calculée sur les acides gras totaux est de 15 à 25% et de préférence de 18,5 à 20,5%.

L'ester d'acide gras ayant au total 40 atomes de carbone est avantageusement constitué d'un acide gras choisi dans le groupe formé par les acides suivants :
— acide eicosanedioïque, acide eicosanoïque ou acide arachidique, ayant 20 atomes de carbone,
— acide docosanoïque ou béhénique
— acide cis 13-docoséno que ou acide érucique
— acide trans 13-docosénoïque ou acide isoérucique
— acide 13-docosynoïque ou acide béhénolique ayant 22 atomes de carbone.

Lorsque l'acide gras formant l'ester en $C_{40}$ comporte 20 atomes de carbone, l'alcool sera choisi parmi les alcools suivants ayant également 20 atomes de carbone :
— 1-eicosanol ou alcool arachidique
— 2-eicosanol
— 9-octadécan-1-ol ou élaidyl alcool.

Lorsque l'acide gras de l'ester en $C_{40}$ comporte 22 atomes de carbone l'alcool sera avantageusement choisi parmi les alcools ci-après ayant 18 atomes de carbone :
— octadécan-1-ol ou alcool stéarylique
— cis 9-octadécan-1-ol ou alcool oléylique
— trans 9-octadécan-1-ol ou alcool élaidylique

On préfère un ester d'acide gras constitué d'un acide ayant 22 atomes de carbone et d'un alcool ayant 18 atomes de carbone.

La chelonine contient avantageusement de 0,01 à 1,0% et de préférence de 0,1 à 0,5% de vitamines choisie dans le groupe formé par les vitamines A, E, F, H, D. Elle contient également avantageusement un antioxydant en une quantité comprise entre 0,5 et 1,5% du poids total de la composition.

La chelonine se conserve bien, présente une bonne compatibilité avec l'épiderme, elle est légèrement astringente et par conséquent ne dilate pas la peau et communique à celle-ci un toucher doux et agréable.

Une composition avantageuse de la chelonine est la suivante exprimée en en % en poids :
— triglycérides d'acides gras 89%
— ester d'acides gras ayant au total 40 atomes de carbone 10%

— vitamines 0,1 à 0,5%
— antioxydant 0,5 à 1,5%
— total 100%.
    Composition des acides gras des triglycérides :
— acide myristique 0,5%
— acide palmytique 71,9%
— acide stéarique 6,1%
— acide linoléique 9,0%
— acide linolénique 1,1%
— acide oléque 11,35%
— total 100%.
    La chélonine peut être avantageusement préparée dans un réacteur muni d'agitation et de moyens de chauffage et de refroidissement.
    On mélange les triglycérides à chaud entre 35 et 50°C, sans dépasser cette dernière température, sous flux d'azote, tout en agitant. Après fusion des triglycérides on ajoute à une température comprise entre 45 et 50°C l'ester l'acide gras en C$_{40}$. On continue à malaxer tout en laissant refroidir jusqu'à environ 20°C. On ajoute à cette température les vitamines et l'antioxydant et on continue l'agitation à la température ambiante pendant 24 heures. On contrôle la stabilité en déterminant le pourcentage de peroxyde.
    La chelonine est avantageusement utilisée dans diverses cosmétiques pour le traitement des cheveux et de la peau notamment dans les shampooings, les compositions après-shampooings, les laits et les crèmes pour le visage, les mains et le corps, les crèmes anti-solaires, les crèmes anti-rides et les compositions similaires.
    La chelonine est utilisée dans les compositions cométiques en une proportion de 0,05 à 30% et de préférence de 0,1 à 15% en poids du poids total de la composition.
    L'utilisation de la chélonine est illustré par les exemples ci-après dont les pourcentages s'entendent en poids.

EXEMPLE 1

    On prépare un shampooing ayant la composition suivante :
— Monolauréate de sorbitan polyéthoxylé avec 20 moles d'oxyde d'éthylène,
    fabriqué sous la dénomination "TWEEN 20" par ATLAS 10%
— Monooléate de sorbitan polyéthoxylé avec 20 moles d'oxyde d'éthylène,
    fabriqué sous la dénomination "TWEEN 80" par ATLAS 5%
— Esters d'acides gras polyoxyuéthylénés
    fabriqués sous la dénomination "ALATONE 289" par ATLAS 10%
— Chelonine 0,1%
— Solution aqueuse de chlorure d'alkyl diméthyl hydroxyéthyl ammonnium 5%
— 1-alkyl (C$_{12}$) amido-3-diméthyl ammonio-propane-3-carboxyméthyl bétaïne 30%
— Eau 37,6%
— Extrait liquide de feuille d'aloès 2%
— Conservateur 0,2%
— Parfum 0,1%
— Total 100%

EXEMPLE 2

    On prépare un lait corporel ayant la composition suivante et qui se présente sous la forme d'une émulsion huile dans l'eau :
(A)
— Monostéarate de glycérol auto-émulsionnable,
    fabriqué sous la dénomination "ARLACEL 165" par ATLAS 8%
— Chelonine 1%
— Mélange de triglycérides d'acides gras saturés en C$_8$–C$_{10}$ provenant
    de l'huile de coco vendue sous la dénomination "MIGLIOL 812" par DYNA-FRANCE 5%
— Perhydrosqualène 7%
— Huile de sésame 2%
(B)
— Glycérine 5%
— Eau 69,7%
— Extraits végétaux 2%
— Conservateurs 0,15%
— Parfums 0,15%

EXEMPLE 3

On prépare la crème anti-solaire ayant la composition ci-après et qui se présente sous la forme d'une émulsion eau dans huile :

(A)
– Vaseline avec des alcools de lanoline émulsifiants 45%
– Hydroxy-2-méthoxy-4-benzophénol, fabriqué sous la dénomination "EUSOLEX 4360" par MERCK 3%
– 4-tert. butyl-4'-méthoxy-dibenzoylméthane,
    fabriqué sous la dénomination "PARSOL 1789" par GIVAUDAN 1%
– Chelonine 5%
– Butyl hydroxy toluène-palmitate d'ascorbine-acide citrique,
    fabriqué sous la dénomination "OXYNEX 2004" par MERCK 0,04%
(B)
– Sorbitol F liquide 3%
– Eau 42,93%
– Conservateur, quantité suffisante
– Parfum, quantité suffisante
– Total 100%

EXEMPLE 4

On prépare une crème anti-rides ayant la composition suivante et qui se présente sous la forme d'une émulsion huile dans l'eau :

(A)
– Mélange de triglycérides d'acides gras saturés en $C_8$–$C_{10}$ provenant du fractionnement
    de l'huile de coco, vendu sous la dénomination "MIGLIOL 812" par DYNA-FRANCE 6%
– 4-Tert. butyl-4'-méthoxy-dibenzoylméthane vendu
    sous la dénomination "PARSOL 1789" par GIVAUDAN 2%
– Chelonine 14%
– Perhydrosqualène 3,98%
– Huile de sésame 2%
– Monostéarate de glycérol auto-émulsionnable,
    fabriqué sous la dénomination "ARLACEL 165" par ATLAS 8%
– Monostéarate de sorbitan polyéthoxylé avec 20 moles d'oxyde d'éthylène,
    fabriqué sous la dénomination "TWEEN 60" par ATLAS 2%
– Alcool cétylique 2%
(B)
– Eau 49,97%
– Complexe hydrant (NMF) 3%
– Hyaluronate de sodium 0,2%
– Extraits de cellules de peau 1%
– Extraits de cellules de foie 1%
– Extrait liquide de feuilles d'aloès 2%
– Collagène ... 3%
– Conservateur en quantité suffisante
– Parfums en quantité suffisante
– Total 100%

**Revendications**

1. Composition de corps gras végétale pour usage cosmétique, caractérisée en ce que
(i) elle comprend en % en poids du poids total de la composition :
    – triglycérides d'acides gras 85 à 94%
    – ester d'acides gras ayant au total 40 atomes de carbone 7 à 14%
    – vitamines A, E, F, H ou D 0,01 à 1,0%
    – antioxydant 0,5 à 1,5%
(ii) la composition moyenne des acides gras des triglycérides, exprimée en % en poids est le suivant :
    – acide myristique 0,35–0,7%
    – acide palmytique 68–76%
    – acide stéarique 4– 7,5%
    – acide linoléique 8–10,5%
    – acide linolénique 0,5–2%
    – acide oléique 8–14%

4

2. Composition selon la revendication 1, caractérisée en ce que :
(i) elle comprend en % en poids du poids total de la composition :
   – triglycérides d'acides gras 88-92%
   – esters d'acides gras ayant au total 40 atomes de carbone 8,5–11,5%
   – vitamines 0,5–0,5%
   – Antioxydant 0,5–1,5%

3. Composition selon la revendication 1 ou2, caractérisée par le fait que la teneur en esters d'acides gras ayant au total 40 atomes de carbone est de 9 à 11% en poids du poids total de la composition.

4. Composition selon la revendication 3, caractérisée par le fait que la teneur en esters d'acides gras ayant au total 40 atomes de carbone est de 10% du poids total de la composition.

5. Composition selon l'une quelconque des revendications 1 à 4, caractérisée par le fait que la composition moyenne des acides gras des triglycérides exprimée en % en poids est la suivante :
   – acide myristique 0,45–0,5%
   – acide palmytique 70–74%
   – acide stéarique 5,5– 6,7%
   – acide linoléique 8,5– 9,5%
   – acide linolénique 0,6– 1,6%
   – acide oléique 10,3–12,3%

6. Composition selon la revendication 5, caractérisée par le fait que la composition moyenne des acides gras des triglycérides, exprimée en % en poids est la suivante :
   – acide myristique 0,55%
   – acide palmytique 71,9%
   – acide stéarique 6,1%
   – acide linoléique 9%
   – acide linolénique 1,1%
   – acide oléique 11,35%

7. Composition selon l'une quelconque des revendications 1 à 6, caractérisée par le fait que dans les triglycérides d'acides gras la proportion des acides gras insaturés calculée sur les acides gras totaux est de 15 à 25%.

8. Composition selon la revendication 7, caractérisée par le fait que dans les triglycérides d'acides gras la proportion des acides gras insaturés calculée sur les acides gras totaux est de 18,5 à 20,5%.

9. Composition cosmétique contenant une composition de corps gras selon l'une quelconque des revendications 1 à 8.

**Claims**

1. Vegetable glyceride composition for cosmetic use, characterized in that
(i) it comprises, as % by weight of the total weight of the compositon:
   – fatty acid triglycerides 85 to 94%
   – fatty acid ester having in total 40 carbon atoms, 7 to 14%
   – vitamins A, E, F, H and D 0.01 to 1.0%
   – antioxidant 0.5 to 1,5%
(ii) the average composition of the fatty acids of the triglycerides, expressed as % by weight, is the following:
   – myristic acid 0.35–0.7%
   – palmitic acid 68–76%
   – stearic acid 4–7.5%
   – linoleic acid 8–10.5%
   – linoleic acid 0.5–2%
   – oleic acid 8–14%

2. Composition according to Claim 1, characterized in that:
(i) it comprises, as % by weight of the total weight of the composition:
   – fatty acid triglycerides 88–92%
   – fatty acid esters having in total 40 carbon atoms 8.5–11.5%
   – Vitamins 0.1–0.5%
   – Antioxidant 0.5–1.5%

3. Composition according to Claim 1 or 2, characterized in that the content of fatty acid esters having in total 40 carbon atoms is from 9 to 11% by weight of the total weight of the composition.

4. Composition according to Claim 3, characterized in that the content of fatty acid esters having in total 40 carbon atoms is 10% of the total weight of the composition.

5. Composition according to any one of Claims 1 to 4, characterized in that the average composition of the fatty acids of the triglycerides, expressed as % by weight, is the following:
   – myristic acid 0.45–0.5%
   – palmitic acid 70–74%

– stearic acid 5.5–6.7%
– linoleic acid 8.5–9.5%
– linolenic acid 0.6–1.6%
– oleic acid 10.3–12.3%

6. Composition according to Claim 5, characterized in that the average composition of the fatty acids of the triglycerides, expressed as % by weight, is the following:
– myristic acid 0.55%
– palmitic acid 71.9%
– stearic acid 6.1%
– linoleic acid 9%
– linolenic acid 1.1%
– oleic acid 11.35%

7. Composition according to any one of Claims 1 to 6, characterized in that within the fatty acid triglycerides the proportion of unsaturated fatty acids, calculated with respect to the total fatty acids, is from 15 to 25%.

8. Composition according to Claim 7, characterized in that within the fatty acid triglycerides the proportion of unsaturated fatty acids, calculated with respect to the total fatty acids, is from 18.5 to 20.5%.

9. Cosmetic composition containing a glyceride composition according to any of Claims 1 to 8.


**Patentansprüche**

1. Fettzusammensetzung pflanzlichen Ursprungs zur kosmetischen Verwendung, dadurch gekennzeichnet, daß
(i) sie in Gewichtsprozent, bezogen auf das Gesamtgewicht der Zusammensetzung, enthält:
– Fettsäuretriglyceride 85–94%
– Fettsäureester mit zusammen 40 Kohlenstoffatomen 7–14%
– Vitamine A, E, F, H, D 0,01–1,0%
– Antioxidationsmittel 0,5–1,5%
(ii) daß die mittlere Zusammensetzung der Fettsäure der Triglyceride, ausgedrückt in Gewichtsprozent, die folgende ist:
– Myristinsäure 0.35–0.7%
– Palmytinsäure 68–76%
– Stearinsäure 4–7,5%
– Linolsäure 8–10,5%
– Linolensäure 0,5–2%
– Oleinsäure 8–14%

2. Zusammensetzung nach Anspruch 1, dadurch gekennzeichnet, daß:
(i) sie in Gewichtsprozent, bezogen auf das Gesamtgewicht der Zusammensetzung, enthält:
– Fettsäuretriglyceride 88–92%
– Fettsäureester mit insgesamt 40 Kohlenstoffatomen 8,5–11,5%
– Vitamine 0,1–0,5%
– Antioxidationsmittel 0,5–1,5%

3. Zusammensetzung nach Anspruch 1 oder 2, dadurch gekennzeichnet, daß der Gehalt an Fettsäureestern mit insgesamt 40 Kohlenstoffatomen zwischen 9 bis 11 Gew.-%, bezogen auf das Gesamtgewicht der Zusammensetzung, liegt.

4. Zusammensetzung nach Anspruch 3, dadurch gekennzeichnet, daß der Gehalt an Fettsäureestern mit insgesamt 40 Kohlenstoffatomen 10 Gew.-%, bezogen auf das Gesamtgewicht der Zusammensetzung, beträgt.

5. Zusammensetzung nach einem der Ansprüche 1 bis 4, dadurch gekennzeichnet, daß die mittlere Zusammensetzung der Fettsäuren der Triglyceride, ausgedrückt in Gewichtsprozent, die folgende ist:
– Myristinsäure 0,45–0,5%
– Palmytinsäure 70–74%
– Stearinsäure 5,5–6,7%
– Linolsäure 8,5–9,5%
– Linolensäure 0,6–1,6%
– Oleinsäure 10,3–12,3%

6. Zusammensetzung nach Anspruch 5, dadurch gekennzeichnet, daß die mittlere Zusammensetzung der Fettsäuren der Triglyceride, ausgedrückt in Gewichtsprozent, die folgende ist:
– Myristinsäure 0,55%
– Palmytinsäure 71,9%
– Stearinsäure 6,1%
– Linolsäure 9%
– Linolensäure 1,1%
– Oleinsäure 11,35%

7. Zusammensetzung nach einem der Ansprüche 1 bis 6, dadurch gekennzeichnet, daß bei den Fettsäuretriglyceriden das Verhältnis der ungesättigten Fettsäuren gegenüber den Gesamtfettsäuren zwischen 15 und 25% liegt.

8. Zusammensetzung nach Anspruch 7, dadurch gekennzeichnet, daß bei den Fettsäuretriglyceriden das Verhältnis der ungesättigten Fettsäuren gegenüber den gesamten Fettsäuren zwischen 18,5 und 20,5% liegt.

9. Kosmetische Zusammensetzung, enthaltend eine Fettzusammensetzung nach einem der Ansprüche 1 bis 8.